Europäisches Patentamt

European Patent Office

Office européen des brevets

(11) Veröffentlichungsnummer: **0 169 493**

**A1**

(12) **EUROPÄISCHE PATENTANMELDUNG**

(21) Anmeldenummer: **85108932.6**

(22) Anmeldetag: **17.07.85**

(51) Int. Cl.⁴: **A 61 M 5/30**

(30) Priorität: **24.07.84 DE 3427189**

(43) Veröffentlichungstag der Anmeldung:
**29.01.86 Patentblatt 86/5**

(84) Benannte Vertragsstaaten:
**CH DE FR GB LI**

(71) Anmelder: **HOECHST AKTIENGESELLSCHAFT**
**Postfach 80 03 20**
**D-6230 Frankfurt am Main 80(DE)**

(72) Erfinder: **Dettbarn, Hans-Jürgen**
**Rehbocks Ecke 4**
**D-3550 Marburg 9(DE)**

(72) Erfinder: **Zimmermann, Josef**
**Auf der Krautweide 11**
**D-6231 Sulzbach(DE)**

(54) **Verriegelung für die abnehmbare Impfstoffpumpe eines nadellosen Injektionsgerätes.**

(57) Bei einem nadellosen Injektionsgerät mit abnehmbarer Impfstoffpumpe (2) ist das Motorgehäuse (1) zur Aufnahme der Impfstoffpumpe (2) mit einer Aufnahmebüchse (20) versehen. In einer Öffnung (7) im Motorgehäuse (1) und der Aufnahmebüchse (20) ist ein mit einer Feder (9) belasteter Riegel (6) angeordnet, der eine Nase (5) aufweist, die in eine Ausnehmung (4) im Pumpengehäuse (18) eingreift. Das Motorgehäuse (1) ist ferner mit einem verschiebbaren Bedienungselement (8) versehen, mit dem der Riegel (6) verbunden ist und das eine Ausnehmung (11) zur Aufnahme einer Arretiereinrichtung (14) aufweist. Die Arretiereinrichtung (14) ist mittels Blattfeder (13) am Motorgehäuse (1) befestigt; sie kann sowohl in eine Rille (15) eingreifen, die sich in einem im Motorgehäuse (1) angeordneten Federgehäuse (17) befindet als auch in die Ausnehmung (11) des Bedienungselementes (8).

EP 0 169 493 A1

./...

FIG.1

Verriegelung für die abnehmbare Impfstoffpumpe eines
nadellosen Injektionsgerätes

Gegenstand der Erfindung ist eine Verriegelung für die abnehmbare Impfstoffpumpe eines nadellosen Injektionsgerätes mit einem federgetriebenen Antriebsmotor für die Impfstoffpumpe, dessen Arbeitskolben mit Federgehäuse verschiebbar in der zylindrischen Bohrung des Motorgehäuses angeordnet ist und einen Kolbenschaft aufweist, das Motorgehäuse zur Aufnahme der Impfstoffpumpe mit einer Aufnahmebüchse versehen ist und das Pumpengehäuse der Impfstoffpumpe mit dem Motorgehäuse und der Pumpenstempel der Impfstoffpumpe mit dem Kolbenschaft lösbar verbunden sind.

Injektionsgeräte der genannten Art sind aus den europäischen Offenlegungsschriften 0 062 916 und 0 063 343 bekannt. Nachteilig ist, daß die Impfstoffpumpe bei gespanntem Arbeitskolben des Antriebsmotors, d.h. in geladenem Zustand des Injektionsgerätes verdreht und/oder abgenommen werden kann.

Hier will die Erfindung Abhilfe schaffen. Die Erfindung, wie sie im Patentanspruch gekennzeichnet ist, löst die Aufgabe dadurch, daß

a) in einer Öffnung im Motorgehäuse und der Aufnahmebüchse ein mit einer Feder belasteter Riegel angeordnet ist, der eine Nase aufweist, die in eine entsprechende Ausnehmung im Pumpengehäuse eingreift,

b) das Motorgehäuse mit einem verschiebbaren Bedienungselement versehen ist, mit dem der Riegel verbunden ist und das eine Ausnehmung zur Aufnahme einer Arretiereinrichtung aufweist,

c) die Arretiereinrichtung mittels Blattfeder am Motorgehäuse gefestigt ist und sowohl in eine im Federgehäuse angeordnete Rille als auch in die Ausnehmung des Bedienungselementes eingreifen kann.

Das Motorgehäuse kann mit einer Schwalbenschwanzführung zur Aufnahme des Bedienungselementes versehen sein.

Im folgenden wird die Erfindung anhand von lediglich einen Ausführungsweg darstellenden Zeichnungen näher erläutert. Es zeigt:

Figur 1 eine Seitenansicht des Injektionsgerätes teilweise geschnitten in geladenem Zustand und verriegelt

Figur 2 einen Ausschnitt der Seitenansicht des Injektionsgerätes gemäß Figur 1 in ungeladenem Zustand und entriegelt.

Das nadellose Injektionsgerät besteht im wesentlichen aus einer Impfstoffpumpe 2 für das zu injizierende Medium, die mit einem Antriebsmotor 3 lösbar verbunden ist. Die Impfstoffpumpe 2 trägt die Einrichtung 24 für den Impfstoffbehälter 25. Der Arbeitskolben 26 des Antriebsmotors 3 ist in einer zylindrischen Bohrung 27 des Motorgehäuses 1 angeordnet, ebenso die Aufnahmebüchse 20 für die Impfstoffpumpe 2. Der Arbeitskolben 26 weist ein Federgehäuse 17 auf, in dem die Antriebsfeder 28 für den Arbeitskolben 26 angeordnet ist. Das Pumpengehäuse 18 ist mit dem Motorgehäuse 1 via Aufnahmebüchse 20 und der Pumpenstempel 10 mit dem Kolbenschaft 21 des Arbeitskolbens 26 lösbar verbunden. Das Motorgehäuse 1 und die Aufnahmebüchse 20 weisen eine Öffnung 7 auf, in der ein Riegel 6 angeordnet ist. Der Riegel 6 wird in Richtung A durch eine Feder 9 belastet, die in einer Bohrung 23 in der Aufnahmebüchse 20 angeordnet ist. Der Riegel 6 weist eine Nase 5 auf, die in eine entsprechende Ausnehmung 4 im Pumpengehäuse 18 eingreifen kann. Das Motorgehäuse 1 ist mit einem in Pfeilrichtung A und B verschiebbaren Bedienungselement 8 versehen, mit dem der Riegel 6 verbunden ist. Das Bedienungselement 8, das in einer Schwalbenschwanzführung (nicht dargestellt) geführt sein kann, weist eine Ausnehmung 11 mit einer Schräge 12 zur Aufnahme einer Arretiereinrichtung 14 auf. Die Arretiereinrichtung 14 ist mittels Blattfeder 13 am Motorgehäuse 1 befestigt, das an dieser Stelle mit einem Durchbruch 19 versehen ist, damit die Arretiereinrichtung 14 sowohl in die Ausnehmung 11 des Bedienungselementes 8 als auch in eine im Federgehäuse 17 angeordnete Rille 15 eingreifen kann.

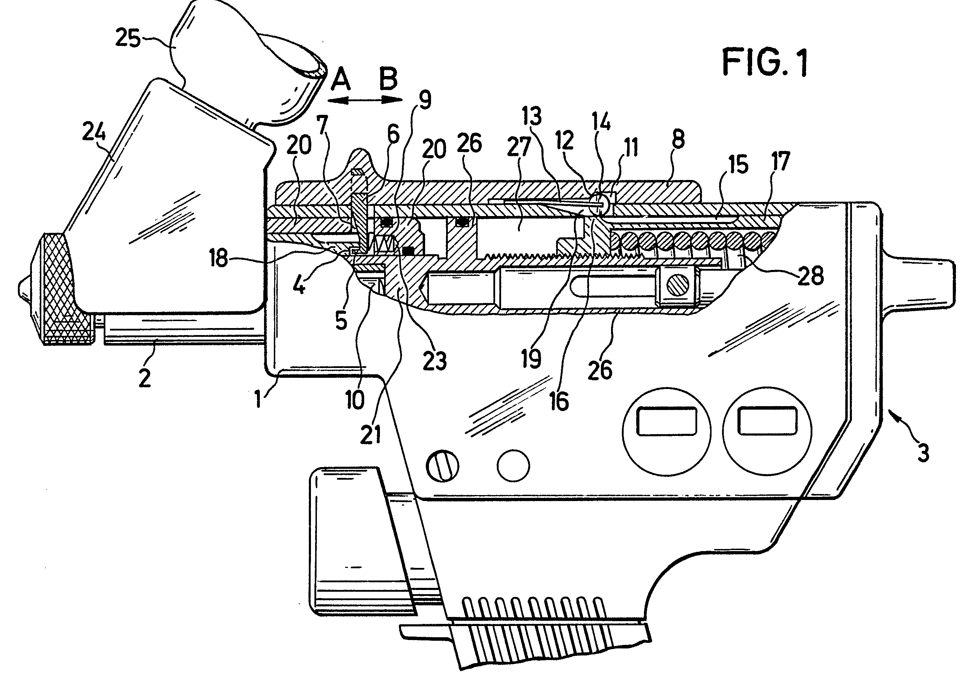

Bei gespanntem Injektionsgerät (Figur 1) kommt die Erhebung 16 des Federgehäuses 17 unter der Arretiereinrichtung 14, z.B. einer Rolle oder einer Kugel zu liegen. Dadurch wird die Arretiereinrichtung 14 in der Ausnehmung 11 gehalten, so daß ein Verschieben des Bedienungselementes 8 gegenüber dem Motorgehäuse 1 unmöglich ist. Die Impfstoffpumpe 2 kann weder verdreht noch abgekuppelt werden (verriegelt).

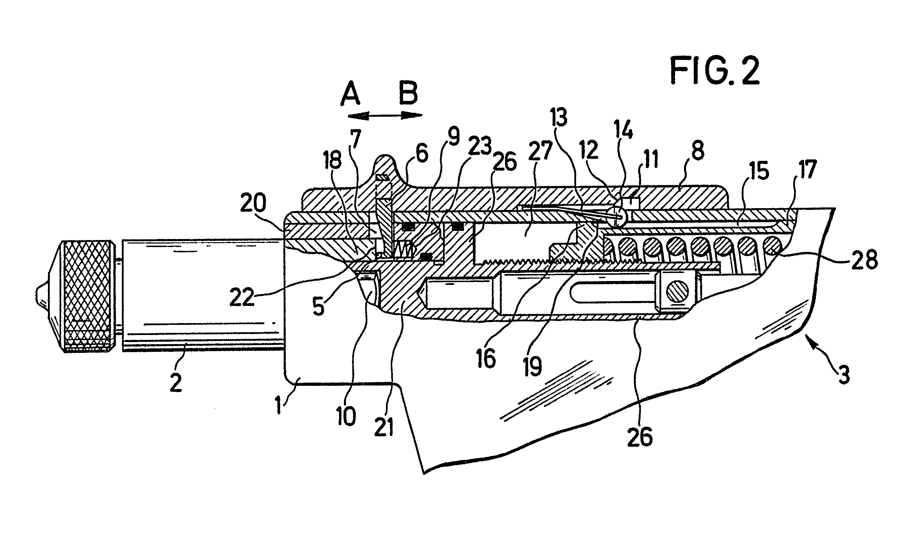

Bei ungespanntem Injektionsgerät (Figur 2) kommt die Rille 15 des Federgehäuses 17 unter der Arretiereinrichtung 14 zu liegen. Beim Zurückschieben des Bedienungselementes 8 in Pfeilrichtung B drückt die Schräge 12 die Arretiereinrichtung 14 in die Rille 15 des Federgehäuses 17. Das Bedienungselement 8 kann hin und her geschoben, die Impfstoffpumpe gedreht und abgekoppelt werden (entriegelt).

Beim Ankoppeln wird die Impfstoffpumpe 2 um ihre Längsachse um 90° gegenüber der Normallage gedreht und in die Aufnahmebüchse 20 des Antriebsmotors 3 geschoben.

Hierbei drückt die rückseitige Planfläche 22 des Pumpengehäuses 18 gegen die Nase 5 des Riegels 6 und schiebt den Riegel und das an ihm befestigte Bedienungselement 8 gegen die Kraft der Feder 9 in Richtung des Pfeiles B. Anschließend wird die Impfstoffpumpe 2 in ihre Normallage gedreht, so daß die Nase 5 in die Ausnehmung 4 im Pumpengehäuse 18 einrasten kann. Beim Einrasten werden das Bedienungselement 8 und der Riegel 6 von der Feder 9 in Richtung des Pfeiles A geschoben.

Das Abkuppeln der Impfstoffpumpe 2 kann wie bereits gesagt, nur im ungeladenen Zustand des Injektionsgerätes, also bei entspanntem Zustand der Feder 28 erfolgen. Dabei wird das Bedienungselement 8 und der daran befestigte Riegel 6 von Hand gegen die Kraft der Feder 9 in Richtung des Pfeiles B geschoben und in dieser Lage gehalten. Die Nase 5 des Riegels 6 gibt die Impfstoffpumpe 2 frei, die nun um 90° gegenüber ihrer Normallage gedreht und aus der Aufnahmebüchse 20 gezogen werden kann.

PATENTANSPRÜCHE:

1. Verriegelung für die abnehmbare Impfstoffpumpe eines nadellosen Injektionsgerätes mit einem federgetriebenen Antriebsmotor für die Impfstoffpumpe, dessen Arbeitskolben mit Federgehäuse verschiebbar in der zylindrischen Bohrung des Motorgehäuses angeordnet ist und einen Kolbenschaft aufweist, das Motorgehäuse zur Aufnahme der Impfstoffpumpe mit einer Aufnahmebüchse versehen ist und das Pumpengehäuse der Impfstoffpumpe mit dem Motorgehäuse und der Pumpenstempel der Impfstoffpumpe mit dem Kolbenschaft lösbar verbunden sind, dadurch gekennzeichnet, daß

   a) in einer Öffnung (7) im Motorgehäuse (1) und der Aufnahmebüchse (20) ein mit einer Feder (9) belasteter Riegel (6) angeordnet ist, der eine Nase (5) aufweist, die in eine entsprechende Ausnehmung (4) im Pumpengehäuse (18) eingreift,

   b) das Motorgehäuse (1) mit einem verschiebbaren Bedienungselement (8) versehen ist, mit dem der Riegel (6) verbunden ist und das eine Ausnehmung (11) zur Aufnahme einer Arretiereinrichtung (14) aufweist,

   c) die Arretiereinrichtung (14) mittels Blattfeder (13) am Motorgehäuse (1) befestigt ist und sowohl in eine im Federgehäuse (17) angeordnete Rille (15) als auch in die Ausnehmung (11) des Bedienungselementes (8) eingreifen kann.

2. Verriegelung nach Anspruch 1, dadurch gekennzeichnet, daß das Motorgehäuse (1) mit einer Schwalbenschwanzführung zur Aufnahme des Bedienungselementes (8) versehen ist.

FIG.1

# FIG.2

**0169493**

Nummer der Anmeldung

## EUROPÄISCHER RECHERCHENBERICHT

Europäisches Patentamt

| EINSCHLÄGIGE DOKUMENTE | | EP 85108932.6 |
|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 062 916 (HOECHST AG)<br>* Gesamt; Fig. 1-4; Seite 4, Zeile 22 - Seite 5, Zeile 24 *<br>-- | 1 | A 61 M 5/30 |
| D,A | EP - A1 - 0 063 343 (HOECHST AG)<br>* Gesamt; Fig. 1 *<br>---- | 1 | |
| | | | **RECHERCHIERTE SACHGEBIETE (Int. Cl.4)**<br><br>A 61 M 5/00 |

Der vorliegende Recherchenbericht wurde für alle Patentansprüche erstellt.

| Recherchenort | Abschlußdatum der Recherche | Prüfer |
|---|---|---|
| WIEN | 16-10-1985 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82